# EUROPEAN PATENT APPLICATION

(11) **EP 2 886 110 A1**
(43) Date of publication of application: **24.06.2015**
(21) Application number: 14197896.5
(22) Date of filing: 15.12.2014
(51) Int. Cl.: A61K 9/50, A61K 31/00

(54) **Multi-layered, multiple unit pharmaceutical compositions**

(30) Priority: 23.12.2013 US 201314138856
(71) Applicant: Ranbaxy Laboratories Limited, New Delhi 110019 (IN)
(72) Inventor: Bhavarisetti, Murali Krishna, 521185 Krishna, Andhra Pradesh (IN); Bakshi, Kanwarpreet Singh, 122001 Gurgaon, Haryana (IN); Narravula, Sreekanth, 522201 Gutur, Andhra Pradesh (IN); Singh, Romi Barat, 221002 Varanasi, Uttar Pradesh (IN); Singla, Ajay Kumar, 122018 Gurgaon, Haryana (IN)
(74) Representative: Cronin, Brian Harold John

(57) **Abstract**

The present invention relates to pharmaceutical compositions comprising multilayered multiple units and processes for the preparation thereof.

## Description

### Field of the Invention

The present invention relates to pharmaceutical compositions comprising multilayered multiple units and processes for the preparation thereof.

### Background of the Invention

Oral controlled-release formulations provide maximum patient compliance and reduce the frequency of dosing to attain effective therapy. The intention of controlled-release formulations is to provide an extended duration of the pharmacological response after administration of the dosage form, longer than is ordinarily experienced after the administration of an immediate-release dosage form. The purpose of these formulations is to provide a constant concentration of the active substance in body fluids for a certain time period. However, the demand on controlled-release dosage forms is immense, the maximal therapeutic effect is to be reached using a minimum amount of active substance with reduced frequency of dosing and lesser degree of side effects, as well as minimized inter- and intra-individual effect variations. The dosage form could be a single unit or multiple unit dosage form.

Single unit controlled-release dosage forms of drugs have known disadvantages. Such dosage forms either pass non-disintegrated through the gastrointestinal tract or release the entire drug in a burst (dose dumping). These dosage forms are dependent upon gastric emptying rates and transit times and are also associated with a lot of intra- and inter-individual variations.

Multiple unit dosage forms comprise a multiplicity of individual units contained within a rapid dissolving capsule, or compressed into a tablet, and soon after ingestion upon its dissolution are available as individual units in the gastrointestinal tract.

Several advantages with multiple unit dosage forms comprising a large number of small units have been described in the literature. It is, for example, possible to obtain a reproducible emptying of the units from the stomach into the small intestine when the particles are less than 1-2 mm. Dispersion over a large area in the gastrointestinal tract can give a more reproducible time for passage, which is of advantage for the absorption process. In addition, a multiple unit preparation is preferable to one single drug unit as the dose is spread out in the intestine. The risk of local irritation and accumulation of several doses due to constriction in the alimentary canal are also considered to be lower.

U.S. Patent Nos. 4,927,640 and 5,246,714 describe controlled-release insoluble beads coated with a membrane controlling drug release. Examples of insoluble inert materials used are silicon dioxide, glass, or plastic resin particles. The core materials have a standardized size and shape, preferably spherical with an even surface with size of 0.15 - 0.25mm. The preparation has several advantages, e.g., the particles contain a high percentage of active ingredient and are not contaminated by soluble inert compounds, which is the case when cores of, *e.g.,* lactose or sugar are covered by a therapeutically active compound. By using small dense particles of, *e.g.,* silicon dioxide as the core material, it is possible to obtain highly concentrated beads (granules) of the active compound which is an advantage for high dosage preparations, e.g., magnesium chloride.

Dosage forms containing multiple layers have several advantages over the prior discussed arts. For example, U.S. Patent No. 5,783,215 describes a multiple unit dose preparation capable of withstanding the mechanical stress, *i.e.,* during compaction. This has been done by using inert and non-soluble cores of glass or sand particles or soluble cores such as sugar spheres capable of withstanding mechanical stress, in combination with a plasticizing layer. The active substance is dispersed in a solution of a hydrophilic polymer and applied to the core, which is again covered with a controlled-release membrane. These beads have excellent mechanical and release characteristics.

PCT Publication No. WO 2004/105735 refers to a controlled-release composition containing units, wherein each unit includes a core, a first layer, and a second layer. In this application it has been disclosed that an inert core (soluble, swellable or insoluble) is first layered with an active ingredient and one or more hydrophilic polymers, and then is further layered with one or more polymers that are effective for controlled-release of the active ingredient.

U.S. Patent No. 8,110,226 discloses a controlled-release drug composition comprising a bead. The bead comprises an inert core; a seal layer positioned on the core layer with the seal layer comprising a non-polymeric hydrophobic material; a layer containing at least one active ingredient positioned on the seal layer; and a layer of at least one release-controlling polymer positioned on the layer containing at least one active ingredient. The seal layer does not contain any polymeric material.

PCT Publication No. WO 2012/101653 discloses a modified-release pharmaceutical composition comprising: (a) a plurality of sustained-release components comprising memantine and one or more rate controlling polymers; (b) at least one immediate-release component comprising memantine coated over the sustained-release components; and (c) more than 3% by weight of one or more pharmaceutically acceptable binders. The composition exhibits a biphasic release profile.

U.S. Patent No. 5,229,135 discloses a sustained-release diltiazem pellet formulation having a central inactive sphere; a plurality of alternating first and second layers surrounding the sphere to form a core, the first layer comprising a water-soluble pharmaceutically acceptable polymeric material and the second layer comprising diltiazem; and an outer coating comprising first inner membrane layers applied to the core. In the formulation, the first inner membrane layers comprising a first water-insoluble pharmaceutically acceptable polymer, and a single outer membrane forming a relatively thick and homogeneous layer surrounding the first inner membrane layers and comprising a second water-insoluble pharmaceutically acceptable polymeric material that is different from the first water-insoluble pharmaceutically acceptable polymer.

Applying a polymer layer over the inert core before the active layer is applied offers advantages. For examples, the amount of time that the solution within the bead would be saturated with respect to the drug may be maximized. Thus, by preventing the soluble core from being a reservoir for drug dissolution, the relative time that a saturated solution would remain within the bead during the release period can be increased considerably. This means that a substantially longer zero order drug-release phase (the phase when the drug-release rate is essentially constant) will be obtained (and less in the undesirable declining release rate phase). By varying the thickness of the first polymeric layer, the drug release profile can be altered in a predictable fashion, in particular for drugs with a moderate to high water solubility.

A similar type of dosage form is disclosed in U.S. Patent No. 6,911,217. It describes a bead comprising (i) a core unit of a substantially water-soluble or water-swellable inert material, (ii) a first layer on the core unit of a substantially water-insoluble polymer, (iii) a second layer covering the first layer and containing an active ingredient, and (iv) a third layer of a polymer on the second layer effective for controlled release of the active ingredient. The first layer of water-insoluble polymer is meant to control water penetration into the core. The U.S. '217 patent describes that in order to achieve water penetration into the core, aqueous polymeric dispersions are used in the first and third layers. However, an aqueous based system requires high heat of vaporization that might require lengthy processing times leading to economic disadvantages. In addition to this, multiple units coated with aqueous based polymeric system can easily agglomerate in the coating process due to low inertia and momentum.

Hence, it would be desirable to have a controlled-release composition in the form of multilayered multiple units that will deliver a constant and controlled-release of water soluble drugs. Further, it is desired to have an advantageous process that would be less time consuming and more economical. The processing time for the present formulation would be less due to use of a non-aqueous based system. In the present invention, a combination of hydrophobic and pH dependent components in the polymeric seal coating on the core helps in modulating the drug release.

### Summary of the Invention

In one aspect, the present invention relates to a multilayered, multiple unit composition comprising:
(i) an inert core;
(ii) a first layer on the inert core, comprising:
   (a) at least one hydrophobic polymer or hydrophobic substance; and
   (b) at least one pH-dependent polymer or pH-dependent substance;
(iii) a second layer on the first layer, the second layer comprising at least one active ingredient;
(iv) a third layer on the second layer, the third layer comprising one or more pharmaceutically acceptable polymers effective for controlling or modifying the release of the active ingredient; and
(v) optionally, a fourth layer on the third layer, the fourth layer comprising one or more pharmaceutically acceptable polymers;
wherein the first layer is applied as a solution or dispersion in a non-aqueous based solvent system.

Embodiments of the composition may include one or more following features. For example, a seal layer comprising one or more pharmaceutically acceptable polymers may optionally be applied between the second active layer and the third controlled-release or modified-release layer.

In one embodiment, the hydrophobic polymer or hydrophobic substance present in the first layer amounts to 0.1 % to 20% of the total weight of the composition. Particularly, the amount is 0.1% to 10%, and more particularly, the amount is 0.1% to 5%.

In another embodiment, the pH-dependent polymer or pH-dependent substance present in the first layer amounts to 0.1% to 20% of the total weight of the composition. Particularly, the amount is 0.1% to 10% and more particularly the amount is 0.1 to 5%.

In yet another embodiment, the ratio of the hydrophobic polymer or hydrophobic substance to the pH-dependent polymer or pH-dependent substance in the first layer may be from about 99:1 to 1:99 by weight.

In yet another embodiment, the active ingredient may include, but is not limited to, antiulcers, analgesics, antihypertensives, antibiotics, antipsychotics, anticancer agents, antimuscarinics, diuretics, antimigraines, antivirals, anti-inflammatory agents, sedatives, antidiabetics, antidepressants, antihistaminics, antiparasitics, antiepileptics, anti-Alzheimer's drugs, and lipid lowering drugs. Particularly, the active ingredient is an antimuscarinic or an anti-Alzheimer's drug, and more particularly, the antimuscarinic is tolterodine and its acceptable salts and the anti-Alzheimer's drug is memantine.

In another aspect, the present invention relates to a process for preparing a multilayered multiple unit composition comprising the steps of:
(i) providing an inert core;
(ii) applying a first layer on the inert core, comprising:
   (a) at least one hydrophobic polymer or hydrophobic substance: and
   (b) at least pH-dependent polymer or pH-dependent substance;
(iii) applying a second layer onto the first layer, the second layer comprising at least one active ingredient;
(iv) applying a third layer onto the second layer, the third layer comprising one or more pharmaceutically acceptable polymers effective for controlling or modifying the release of the active ingredient; and
(v) optionally applying a fourth layer onto the third layer, the fourth layer comprising one or more pharmaceutically acceptable polymers;
wherein the first layer is applied as a solution or dispersion or suspension in a non-aqueous based solvent system.

In yet another aspect, the present invention relates to a process for preparing a multilayered multiple unit composition comprising the steps of:
(i) providing an inert core;
(ii) applying a first layer onto the inert core, the first layer comprising:
   (a) at least one hydrophobic polymer or hydrophobic substance; and
   (b) at least one pH-dependent polymer or pH-dependent substance;
(iii) applying a second layer onto the first layer, the second layer comprising at least one active ingredient;
(iv) applying a seal layer onto the second layer, the seal coat comprising one or more pharmaceutically acceptable polymers;
(v) applying a third layer onto the seal layer, the third layer comprising one or more pharmaceutically acceptable polymers effective for controlling or modifying the release of the active ingredient; and
(vi) optionally applying a fourth layer onto the third layer, comprising one or more pharmaceutically acceptable polymers;
wherein the first layer is applied as a solution, dispersion, or suspension in a non-aqueous based solvent system.

In one embodiment, the seal layer onto the second layer may optionally further include one or more organic acids as stabilizers to prevent any inter-reactions between the drug and the release-controlling or modifying layer.

In yet another aspect, the pharmaceutical composition of the present invention relates to a method of treating urinary disorders including overactive urinary bladder.

Embodiments of the composition may include one or more pharmaceutically acceptable excipients that act in one or more capacities as diluents, binders, plasticizers, lubricants, glidants, colorants, or flavoring agents.

The details of one or more embodiments of the inventions are set forth in the description below. Other features and objects of the invention will be apparent from the description and examples.

### Description of the Invention

Formulating a controlled-release pharmaceutical composition for water-soluble drugs in the form of multiple units often is challenging to the formulator. In this invention, the inventors have developed a multilayered multiple unit composition that is robust and stable to deliver the active ingredient in a controlled manner.

The invention relates to a multilayered multiple unit controlled release composition comprising:
(i) an inert core;
(ii) a first layer on the inert core, the first layer comprising:
   (a) at least one hydrophobic polymer or hydrophobic substance; and
   (b) at least one pH-dependent polymer or pH-dependent substance;
(iii) a second layer on the first layer, the second layer comprising at least one active ingredient;
(iv) a third layer onto the second layer, the third layer comprising one or more pharmaceutically acceptable polymers effective for controlling or modifying the release of the active ingredient;
(v) a seal layer between the second and third layer, the seal layer comprising one or more pharmaceutically acceptable polymers; and
(vi) optionally, a fourth layer onto the third layer, the fourth layer comprising one or more pharmaceutically acceptable polymers;
wherein the first layer is applied as a solution, dispersion or suspension in a non-aqueous based solvent system.

The term "multiple unit composition" indicates a pharmaceutical composition that includes one or more individual coated units contained in the formulation in such a form that the individual units will be available from the formulation upon disintegration of the formulation in the stomach. The multiple unit pharmaceutical composition or formulation may be a capsule or a tablet that disintegrates in the stomach to give individual units. The multiple units may be formulated as granules, pellets, or beads.

The inert core of the composition may include one or more of an inert insoluble, swellable, or soluble core. The insoluble or swellable inert core may include one or more of dicalcium phosphate, microcrystalline cellulose, or any of the marketed inert cores, for example, glass beads, silicate beads, sugar spheres, non-pareils, and celphere. The soluble core may include one or more of glucose, mannitol, lactose, xylitol, dextrose, and sucrose.

The first layer of the composition comprises (a) at least one hydrophobic polymer or hydrophobic substance, and (b) at least one pH-dependent polymer or pH-dependent substance.

Suitable examples of hydrophobic polymers or hydrophobic substance include, but are not limited to, ethyl cellulose, cellulose acetate, cellulose acetate butyrate, hydroxypropyl methylcellulose phthalate, poly (alkyl) methacrylate, copolymers of acrylic or methacrylic acid esters, waxes, shellac, hydrogenated vegetable oils, or mixtures thereof. Ethyl cellulose is available as a dry powder (e.g., Ethocel^{®} of Dow, U.S.A.) or as an aqueous dispersion marketed under the trade names Aquacoat^{®} of FMC, USA and Surelease^{®} of Colorcon, USA,. Ethyl cellulose of various available viscosity grades ranging from 3 mpas to 50 mpas can be used.

Suitable examples of pH-dependent polymers or pH-dependent substances include, but are not limited to, cellulose acetate phthalate, polymethacrylates, sodium carboxymethylcellulose, chitosan, sodium alginate, and oleic acid. Polymethacrylate is selected from Poly (methacrylic acid, methyl methacrylate) 1:1 marketed under the trade name of Eudragit^{®} L of Rohm Pharma, Germany, Poly (methacrylic acid, ethyl acrylate) 1:1 marketed under the trade name of Eudragit^{®} L 30D-55 and Eudragit^{®} L100-55 of Rohm Pharma, Germany, Poly(methacrylic acid, methyl methacrylate) 1:2 marketed under the trade name of Eudragit^{®} S of Rohm Pharma, Germany.

The ratio of the hydrophobic polymer or hydrophobic substance to the pH-dependent polymer or pH-dependent substance in the first layer may be from about 99:1 to about 1:99 by weight.

The first layer controls the penetration of water inside the core depending on the pH of the environment. The pH-dependent polymer dissolves at a specific pH and leads to the formation of pores in the first layer leading to water absorption by the osmotic core, which thereby controls the drug-release through a push-pull mechanism.

The second layer of the composition comprises at least one water-soluble active ingredient selected from the group including antiulcers, analgesics, antihypertensives, antibiotics, antipsychotics, anticancer agents, antimuscarinics, diuretics, antimigraines, antivirals, anti-inflammatory agents, sedatives, antidiabetics, antidepressants, antihistaminics, antiparasitics, antiepileptics, anti-Alzheimer's drugs and lipid lowering drugs. The active ingredients are water-soluble or water-insoluble. Particularly, the active ingredient is water-soluble.

Suitable examples of water-soluble active ingredients include, but are not limited to, tolterodine tartrate, memantine, diltiazem hydrochloride, verapamil hydrochloride, bupropion hydrochloride, metformin hydrochloride, propranolol hydrochloride, dextromethorphan hydrobromide, diphenhydramine hydrochloride, disopyramide hydrochloride, tramadol, fluoxetine hydrochloride, paroxetine hydrochloride, pentoxifylline hydrochloride, and the like.

The second layer may additionally comprise a hydrophilic polymer along with the active ingredient that gives plasticity properties to the units and acts as a binder.

Suitable hydrophilic polymers may include, but are not limited to, pharmaceutically acceptable materials like starch, gums, alginates, polysaccharides, polyvinylprrolidone, polyethylene glycol, acrylic acid derivatives, and cellulose derivatives like hydroxypropyl cellulose, hydroxypropyl methylcellulose, hydroxyethylcellulose, hydroxymethylcellulose, carboxymethylcellulose, methylcellulose, sodium carboxy methylcellulose, and mixtures thereof.

The third layer of the composition comprises one or more polymers effective for controlling or modifying the release active ingredient.

The release-controlling polymers may be selected from the group comprising hydrophilic polymers, hydrophobic polymers, or combinations thereof.

Suitable examples of hydrophilic release-controlling polymers include, but are not limited to, cellulose derivatives such as hydroxypropylcellulose, hydroxypropylmethylcellulose, hydroxyethylcellulose, hydroxymethylcellulose, carboxymethylcellulose, methylcellulose, sodium carboxy methylcellulose, or combinations thereof; polyvinylpyrrolidone; polyvinyl acetate; copolymer of vinylpyrrolidone and vinyl acetate; polysaccharides; polyalkylene glycols; starch and derivatives; or mixtures thereof.

Suitable examples of hydrophobic release-controlling polymers include, but are not limited to, ethyl cellulose, cellulose acetate, cellulose acetate butyrate, hydroxypropyl methylcellulose phthalate, poly (alkyl) methacrylate, copolymers of acrylic or methacrylic acid esters, waxes, shellac, and hydrogenated vegetable oils. The hydrophobic release-controlling polymers may be water-based dispersions of ethyl cellulose and is commercially available, for example, as Surelease® and Aquacoat^{®}**.**

The ratio of the hydrophobic polymer to the hydrophilic polymer in the third layer may be from about 99:1 to about 1:99 by weight.

The release modifying polymers may be the enteric polymers and may be selected from any such pharmaceutically acceptable enteric polymers which would facilitate erosion and breakdown of the pellets in the pH of the lower GI tract. These enteric polymers may be selected from the group consisting of cellulose acetate phthalate, hydroxypropyl methylcellulose phthalate, additional cellulose ether phthalates, any of the acrylic acid derivates phthalates (available commercially as Eudragits^{®}), shellac, zein, or mixtures thereof.

The third layer of release-controlling or modifying layer may also include one or more release regulators which are hydrophilic or having pH dependent solubility. The release regulators may include, but are not limited to, hydroxypropyl methylcellulose, hydroxyethyl cellulose, hydroxypropyl cellulose, methylcellulose, carboxymethylcellulose, polyethylene glycol, polyvinylpyrrolidone, polyvinyl alcohol, polymers with pH-dependent solubility such as cellulose acetate phthalate or ammonio-methacrylate copolymer and methacrylic acid copolymer, or mixtures thereof.

The seal layer between the second and third layers of the composition comprises one or more pharmaceutically acceptable polymers that include, but are not limited to, ethyl cellulose, hydroxypropyl methylcellulose, hydroxypropyl cellulose, methyl cellulose, carboxymethylcellulose, hydroxymethylcellulose, hydroxyethylcellulose, hydroxypropyl methyl phthalate, cellulose acetate, cellulose acetate trimelliatate, cellulose acetate phthalate; waxes such as polyethylene glycol; methacrylic acid polymers such as Eudragit^{®} E, L, S, FS, NE, RL, and RS, or mixtures thereof. Alternatively, commercially available coating compositions comprising film-forming polymers marketed under various trade names, such as Opadry^{®} may also be used for coating.

The seal layer may further include one or more organic acids as stabilizers to prevent any inter-reactions between the drug and the release-controlling or modifying layer.

Suitable examples of organic acids used as stabilizers include, but are not limited to, tartaric acid, lactic acid, salicylic acid, citric acid, acetic acid, gluconic acid, succinic acid, and oxalic acid. Particularly, the organic acid is tartaric acid.

The optional fourth layer on the third layer of the composition comprises one or more pharmaceutically acceptable polymers. The polymers may comprise one or more film forming agents and/or pharmaceutically acceptable excipients.

Examples of film forming agents include, but are not limited to, ethyl cellulose, hydroxypropyl methylcellulose, hydroxypropyl cellulose, methyl cellulose, carboxymethylcellulose, hydroxymethylcellulose, hydroxyethylcellulose, hydroxypropyl methyl phthalate, cellulose acetate, cellulose acetate trimelliatate, and cellulose acetate phthalate; waxes such as polyethylene glycol; methacrylic acid polymers such as Eudragit^{®} RL and RS; or mixtures thereof. Alternatively, commercially available coating compositions comprising film-forming polymers marketed under various trade names, such as Opadry^{®} may also be used for coating.

The composition may further include one or more pharmaceutically acceptable excipients acting in one or more capacities as fillers, binders, plasticizers, lubricants, glidants, colorants, and flavoring agents.

Suitable examples of fillers include, but are not limited to, corn starch, lactose, white sugar, sucrose, sugar compressible, sugar confectioners, glucose, sorbitol, calcium carbonate, calcium phosphate-dibasic, calcium phosphate-tribasic, calcium sulfate, microcrystalline cellulose, silicified microcrystalline cellulose, cellulose powdered, dextrates, dextrins, dextrose, fructose, kaolin, lactitol, mannitol, sorbitol, starch, starch pregelatinized, and mixtures thereof.

Examples of binders include, but are not limited to, methyl cellulose, hydroxypropyl cellulose, hydroxypropyl methylcellulose, polyvinylpyrrolidone, poloxamer, gelatin, gum arabic, ethyl cellulose, polyvinyl alcohol, pullutan, pregelatinized starch, agar, tragacanth, sodium alginate, propylene glycol, and mixtures thereof.

Examples of plasticizers include, but are not limited to, propylene glycol, triethylene glycol, oleic acid, ethyleneglycolmonoleate, triethyl citrate, triacetin, diethyl phthalate, glycerylmonostearate, dibutylsebacate, acetyl triethylcitrate, castor oil, medium chain triglycerides, and mixtures thereof.

Examples of lubricants and glidants include, but are not limited to, colloidal anhydrous silica, stearic acid, magnesium stearate, calcium stearate, talc, hydrogenated castor oil, sucrose esters of fatty acids, microcrystalline wax, yellow beeswax, white beeswax, and mixtures thereof.

The coloring agents of the present invention may be selected from any FDA approved colors for oral use.

The non-aqueous solvents used for the preparation of the solution, dispersion, or suspension may include, but are not limited to, alcohols such as ethyl alcohol and isopropyl alcohol; ketones such as acetone and ethylmethylketone; halogenated hydrocarbons such as dichloroethane and trichloroethane; and mixtures thereof. The non-aqueous solvent based system includes completely non-aqueous solvents (for example, a solvent system comprising organic solvents, inorganic solvents, or mixture of both). The non-aqueous solvent based system also includes a substantially non-aqueous solvent comprising at most 20% by weight of water. The remainder of the solvent *(i.e.,* at least 80% by weight) is non-aqueous.

The pharmaceutical compositions of the present invention comprising multilayered multiple units may provide a controlled, extended, or pulsatile delivery.

The coating of the layers may be done using a conventional coating pan, a spray coater, a rotating perforated pan, or an automated system, such as a centrifugal fluidizing (CF) granulator, a fluidized bed process, or any other suitable automated coating equipment.

The coated multiple units are filled into hard gelatin capsules or compressed into tablets that disintegrate in the stomach to make available a multiplicity of individually coated units.

The pharmaceutical composition of the present invention relates to a method of treating urinary disorders including overactive urinary bladder.

The overactive bladder condition gives rise to urinary frequency, urgency, and/or urge incontinence. Overactive bladder disorders also include nocturia, *i.e.,* awakening at night to urinate. While overactive bladder is often associated with detrusor muscle instability, disorders of bladder function may also be due to neuropathy of the central nervous system (detrusor hyperreflexia) including spinal cord and brain lesions, such as multiple sclerosis and stroke. Overactive bladder symptoms may also result from, for example, male bladder outlet obstruction (usually due to prostatic hypertrophy), interstitial cystitis, local edema and irritation due to focal bladder cancer, radiation cystitis due to radiotherapy to the pelvis, and cystitis. The compounds also have spasmolytic activity and may be useful for treating gastrointestinal disorders, including gastrointestinal hyperactivity.

The present invention is illustrated below by reference to the following examples. However, one skilled in the art will appreciate that the specific methods and results discussed are merely illustrative of the invention and not to be construed as limiting the invention.

### Examples

### Example 1:

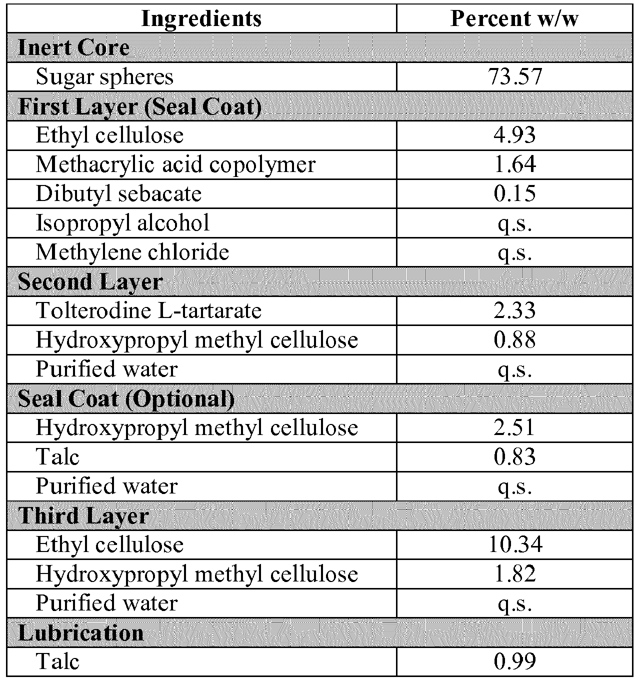

### Procedure:

1. Ethyl cellulose and methacrylic acid copolymer were dispersed in isopropyl alcohol with stirring, and methylene chloride was added to this dispersion to form a clear solution.
2. Dibutyl sebacate was added to the solution of step 1 and this solution was coated over sugar spheres, to form the first layer
3. Tolterodine L-tartarate and hydroxypropyl methyl cellulose were dissolved in water and sprayed over the cores of step 2 to form the second layer.
4. Hydroxypropyl methyl cellulose and talc were dispersed in water and coated over the cores of step 3 to form an optional seal coat.
5. Ethyl cellulose and hydroxypropyl methyl cellulose were dispersed in water and coated over the cores of step 4 to form the third layer.
6. The coated beads of step 5 were dried, lubricated with talc, and filled into capsules.

The capsules of Example 1 were subjected to stability studies at 40°C and 75% relative humidity (RH) for a period of three months. The results are provided in Table 1. The stability data demonstrates that the capsules according to Example 1 are stable.

**Table 1**

| **Total Related substances (% w/w)** | | **Assay (% w/w)** | |
|---|---|---|---|
| **Initial** | **After 3 months at 40 °C and 75 % relative humidity (RH)** | **Initial** | **After 3 months at 40 °C and 75 % relative humidity (RH)** |
| 0.14 | 0.27 | 100.6 | 99.5 |

The capsules of Example 1 also were subjected to dissolution studies in 900 mL of phosphate buffer (pH 6.8) at 37°C ± 0.5°C, using USP apparatus I. The results of initial dissolution and dissolution after storage at 40°C and 75% relative humidity (RH) for the period of 3 months are provided in Table 2. The dissolution data demonstrates that storage at accelerated conditions has minimal impact on dissolution for the tablets made according to Example 1.

**Table 2**

| **Time (in hours)** | **% Drug dissolved** | |
|---|---|---|
| | **Initial** | **After 3 months at 40 °C and 75 % relative humidity (RH)** |
| 1 | 21 | 28 |
| 3 | 62 | 67 |
| 7 | 91 | 90 |

### Example 2:

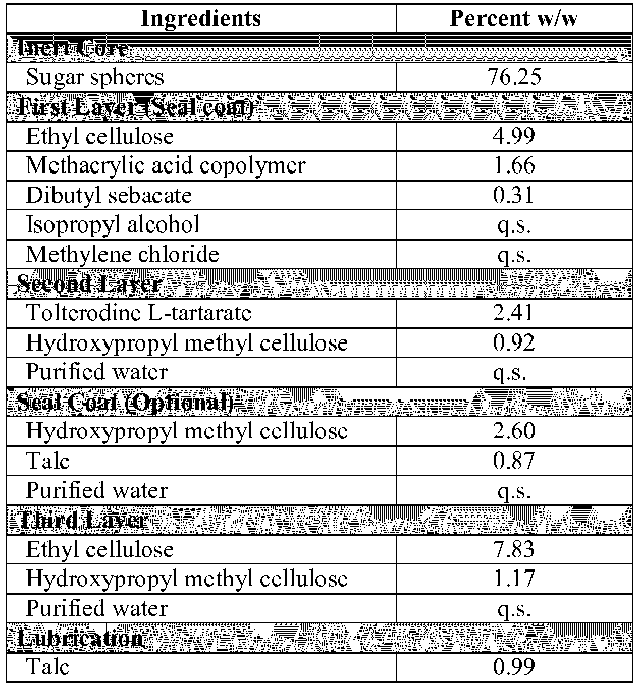

### Procedure:

1. Ethyl cellulose and methacrylic acid copolymer were dispersed in isopropyl alcohol with stirring, and methylene chloride was added to this dispersion to form a clear solution.
2. Dibutyl sebacate was added to the solution of step 1 and this solution was coated over sugar spheres, to form the first layer.
3. Tolterodine L-tartarate and hydroxypropyl methyl cellulose were dissolved in water and sprayed over the cores of step 2, to form the second layer.
4. Hydroxypropyl methyl cellulose and talc were dispersed in water and coated over the cores of step 3, to form an optional seal coat.
5. Ethyl cellulose and hydroxypropyl methyl cellulose were dispersed in water and coated over the cores of step 4, to form the third layer.
6. The coated beads were dried, lubricated with talc, and filled into capsules.

### Example 3:

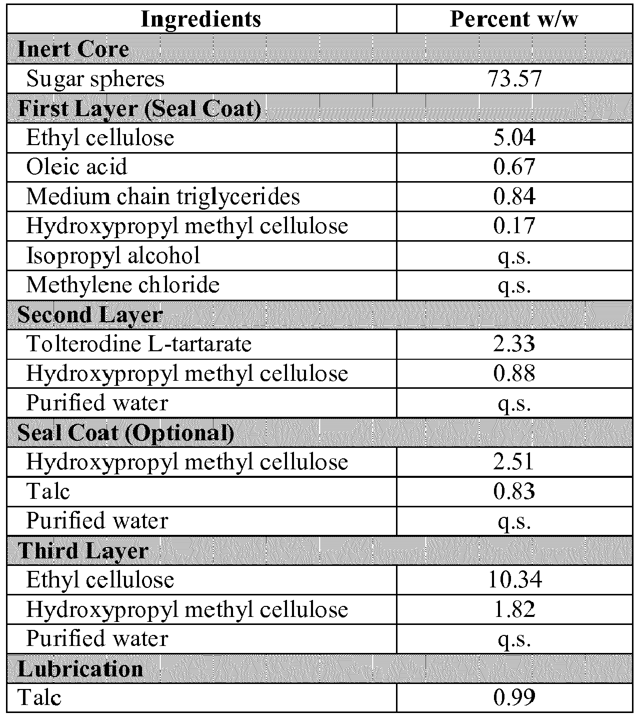

### Procedure:

1. Ethyl cellulose, oleic acid, hydroxypropyl methyl cellulose, and medium chain triglycerides were dispersed in isopropyl alcohol with stirring, then methylene chloride was added to this dispersion to form a clear solution.
2. The solution of step 1 was coated over sugar spheres to form the first layer.
3. Tolterodine L-tartarate and hydroxypropyl methyl cellulose were dissolved in water and sprayed over the cores of step 2 to form the second layer.
4. Hydroxypropyl methyl cellulose and talc were dispersed in water and coated over the cores of step 3 to form an optional seal coat.
5. Ethyl cellulose and hydroxypropyl methyl cellulose were dispersed in water and coated over the cores of step 4 to form the third layer.
6. The coated beads were dried, lubricated with talc, and filled into capsules.

While several particular forms of the invention have been illustrated and described, it will be apparent that various modifications and combinations of the invention detailed in the text can be made without departing from the spirit and scope of the invention.

## Claims

1. A multilayered multiple unit composition comprising:
(i) an inert core;
(ii) a first layer on the inert core, the comprising:
(a) at least one hydrophobic polymer or hydrophobic substance; and
(b) at least one pH-dependent polymer or pH-dependent substance;
(iii) a second layer onto the first layer, the second layer comprising at least one active ingredient;
(iv) a third layer onto the second layer, the third comprising one or more pharmaceutically acceptable polymers effective for controlling or modifying the release of the active ingredient; and
(v) optionally, a fourth layer onto the third layer, the fourth layer comprising one or more pharmaceutically acceptable polymers,
wherein the first layer is applied as a solution or dispersion in a non-aqueous based solvent system.

2. The multilayered multiple unit composition according to claim 1, wherein the hydrophobic polymer or hydrophobic substance is present in the first layer in an amount of about 0.1% to 20% of the total weight of the composition.

3. The multilayered multiple unit composition according to claim 1, wherein the pH-dependent polymer or pH-dependent substance is present in the first layer in an amount of about 0.1% to 20% of the total weight of the composition.

4. The multilayered multiple unit composition according to claim 1, wherein the ratio of the hydrophobic polymer or hydrophobic substance to the pH-dependent polymer or pH-dependent substance in the first layer varies from about 99:1 to 1:99 by weight.

5. The multilayered multiple unit composition according to claim 1, wherein a seal layer comprising one or more pharmaceutically acceptable polymers is optionally applied between the second active layer and the third controlled release or modified release layer.

6. The multilayered multiple unit composition according to claim 1, wherein the composition is prepared using a process comprising the steps of:
(i) providing an inert core;
(ii) applying a first layer on the inert core, the first layer comprising:
(a) at least one hydrophobic polymer or hydrophobic substance; and
(b) at least pH-dependent polymer or pH-dependent substance;
(iii) applying a second layer onto the first layer, the second layer comprising at least one active ingredient;
(iv) applying a third layer onto the second layer, the third layer comprising one or more pharmaceutically acceptable polymers effective for controlling or modifying the release of the active ingredient; and
(v) optionally applying a fourth layer onto the third layer, the fourth layer comprising one or more pharmaceutically acceptable polymers,
wherein the first layer is applied as a solution, dispersion or suspension in a non-aqueous based solvent system.

7. The multilayered multiple unit composition according to claim 5, wherein the composition is prepared using a process comprising the steps of:
(i) providing an inert core;
(ii) applying a first layer on the inert core, the first layer comprising:
(a) at least one hydrophobic polymer or hydrophobic substance; and
(b) at least one pH-dependent polymer or pH-dependent substance;
(iii) applying a second layer onto the first layer, the second layer comprising at least one active ingredient;
(iv) applying a seal layer onto the second layer, the seal layer comprising one or more pharmaceutically acceptable polymers;
(v) applying a third layer onto the seal layer, the third layer comprising one or more pharmaceutically acceptable polymers effective for controlling or modifying the release of the active ingredient; and
(vi) optionally applying a fourth layer onto the third layer, the fourth layer comprising one or more pharmaceutically acceptable polymers,
wherein the first layer is applied as a solution, dispersion or suspension in a non-aqueous based solvent system.

8. The multilayered multiple unit composition according to claim 5, wherein the seal layer onto the second layer optionally includes one or more organic acids.

9. The multilayered multiple unit composition according to claim 1, wherein the said composition includes one or more pharmaceutically acceptable excipients which act in one or more capacities as diluents, binders, plasticizers, lubricants, glidants, colorants, or flavoring agents.

10. The multilayered multiple unit composition according to claim 1 for use in treating urinary disorders including overactive urinary bladder.

11. The multilayered multiple unit composition according to claim 1, wherein the active ingredient comprises an antiulcer, analgesic, antihypertensive, antibiotic, antipsychotic, anticancer agent, antimuscarinic, diuretic, antimigraine, antiviral, anti-inflammatory agent, sedative, antidiabetic, antidepressant, antihistaminic, antiparasitic, antiepileptic, anti-Alzheimer's or lipid lowering drug

12. The multilayered multiple unit composition according to claim 1, wherein the active ingredient comprises tolterodine.
